# FASCICULE DE BREVET EUROPEEN

(11) **EP 0 383 645 B1**
(45) Date de publication et mention de la délivrance du brevet: **14.04.1993**
(21) Numéro de dépôt: 90400203.7
(22) Date de dépôt: 24.01.1990
(51) Int. Cl.: C07K 3/22, A61K 37/02

(54) **Procédé de fabrication du facteur antihémophilique (FVIIIc) ayant une très haute pureté.**
Verfahren zur Herstellung von antihämophilem Faktor (Faktor VIII), der eine hohe Reinheit aufweist.
Process to prepare a very pure antihaemophilic factor.

(30) Priorité: 17.02.1989 FR 8902136
(43) Date de publication de la demande: 22.08.1990
(73) Titulaire: ASSOCIATION D'AQUITAINE POUR LE DEVELOPPEMENT DE LA TRANSFUSION SANGUINE ET DES RECHERCHES HEMATOLOGIQUES, F-33035 Bordeaux Cédex (FR)
(72) Inventeur: Dazey, Bernard Jules, F-33000 Bordeaux (FR); Hamsany, Mohamed, F-33000 Bordeaux (FR); Vezon, Gérard Paul, F-33670 Cursan (FR)
(74) Mandataire: Portal, Gérard

(56) Documents cités:
- EP-A- 0 083 483
- EP-A- 0 104 356
- EP-A- 0 144 957
- EP-A- 0 303 329
- EP-A- 0 359 593
- EP-A- 0 383 234
- WO-A-86/04486
- BRITISH JOURNAL OF HAEMATOLOGY, vol. 43, 1979, pages 669-674, Blackwell Scientific Publications; D.E.G. AUSTEN: "The chromatographic separation of factor VIII on aminohexyl sepharose"
- TROMBOSIS & HAEMOSTASIS, vol. 47, 1982, pages 124-127; J.-J. MORGENTHALER: "Chromatography of antihemophilic factor on diaminoalkane- and aminoalkane-derivatized sepharose"

## Description

L'invention concerne essentiellement un procédé de fabrication du Facteur antihémophilique (FVIIIc) ayant une très haute pureté, le Facteur antihémophilique ainsi obtenu ainsi qu'une composition pharmaceutique le contenant.

On connaît déjà dans l'état antérieur de la technique divers procédés de purification du Facteur antihémophilique (FVIIIc). Par exemple, le document WO86/04486 (New York University) décrit une méthode de purification du Facteur antihémophilique utilisant des techniques de chromatographie sur colonne en présence de sucre, d'alcool polyhydrique, d'aminoacide ou de sel (voir page 1, lignes 16 à 23, ainsi que la revendication 1 page 30).

Selon ce procédé antérieur, l'addition de ces additifs comprenant les sucres, les alcools polyhydriques, les aminoacides ou les sels, est réalisée à n'importe quelle étape du procédé, c'est-à-dire soit avant la chromatographie, soit pendant la chromatographie, soit à la fin de la chromatographie.

Il est à noter qu'une telle addition de sucre était largement utilisée dans la technique antérieure puisque, dans le texte même de ce document, on cite aux pages 3 et 4 de nombreux articles relatifs à la purification du Facteur VIIIc par chromatographie. On peut se reporter utilement à ce sujet au document Austen dans Thromb. Haemostas. (Stuttgart) 48 (1) : 46 (1982) qui décrit un procédé de traitement de plasma par chromatographie sur une colonne de Sépharose aminohexyle à l'aide d'une solution tampon de lavage d'acétate de lysine et un gradient salin pour l'élution de la colonne. De même Faure décrit dans J. Chromatog. 257 : 387 (1983) l'utilisation de 1 et 10 % de saccharose dans des tampons d'acétate de lysine pour améliorer le rendement en Facteur VIIIc pendant la chromatographie d'un cryoprécipité sur un gel d'aminohexyle sépharose utilisé dans la colonne de chromatographie. L'emploi de sucre est indiqué par les auteurs comme permettant d'inhiber la formation d'un complexe moléculaire. Ce document correspond à US 4 743 680.

De manière similaire, le document EP-A-383 234 prévoit l'ajout de 5 à 30 % en poids de carbohydrate (en particulier du saccharose) avant l'échange ionique sur la colonne, ce qui modifie grandement les conditions ioniques du procédé.

D'autres documents de l'état de la technique sont par exemple US-A-4 508 709 = EP-A-144 957 ou encore US-A-4 578 218 = DE-A-3 504 385. Un autre procédé antérieur est décrit dans FR-A-2 570 276 qui conduit à la présence d'immunoglobulines d'origine murine qui présentent quelques inconvénients en thérapie humaine bien compréhensibles à l'homme de l'art.

En particulier, le document EP-A-144 957 ARMOUR (= aussi US-A-4 508 709) décrit un procédé de purification du Facteur VIIIc basé sur une adsorption combinée du Facteur VIIIc et du Facteur Willebrand, puis sur une désorption sélective du Facteur VIIIc du Facteur Willebrand en vue d'éliminer le Facteur Willebrand. Ceci constitue une différence fondamentale de procédure par rapport au procédé de l'invention, qui, comme on le verra plus loin, vise principalement à adsorber seulement le Facteur VIIIc, essentiellement sans Facteur Willebrand. En outre, pour l'adsorption du mélange Facteur VIIIc-Facteur Willebrand, ARMOUR utilise une solution d'élution, dite "tampon A", à un pH d'environ 6,8 , mais qui est exempte de CaCl₂ (voir page 5, lignes 6 à 18 et page 6, lignes 14 à 26).

Ensuite, ARMOUR réalise des lavages avec des solutions tampon modifiées contenant 10 millimoles de CaCl₂. ARMOUR souligne que le CaCl₂ est connu dans l'art pour stabiliser le facteur VIIIc, mais ne peut pas être utilisé antérieurement à cause du danger d'activation des facteurs de coagulation conduisant à des caillots de fibrine sur la résine et une dégradation potentielle du Facteur VIIIc (voir page 6, ligne 27 à page 7, ligne 5).

Ainsi, il existait un préjugé dans l'art contre l'utilisation de solutions initiales d'élution contenant du CaCl₂.

Or, et comme on pourra l'observer plus loin, la présente invention va à l'encontre de ce préjugé dans l'art en visant à adsorber sélectivement le Facteur VIIIc essentiellement dépourvu de Facteur Willebrand et en utilisant pour se faire une première solution d'élution qui contient du CaCl₂ à une quantité de 10 millimoles.

La présente invention a donc pour but de résoudre le nouveau problème technique consistant en la fourniture d'un procédé de fabrication du Facteur antihémophilique (FVIIIc) ayant une très haute pureté, sans immunoglobulines murines, ne comportant pas de contaminants pouvant représenter un désagrément pour le patient hémophile.

La présente invention a encore pour but de résoudre le nouveau problème technique consistant en la fourniture d'un procédé de fabrication de Facteur antihémophilique (FVIIIc) isolé en majeure partie du Facteur Willebrand, contrairement à certaines techniques antérieures et notamment celle décrite dans FR-A-2 570 276, ou EP-A-144 957 (= US-A-4 508 709), en permettant ainsi d'accroître de manière significative l'activité du Facteur antihémophilique ainsi obtenu jusqu'à des valeurs aussi élevées que 250 unités internationales (U.I.) par mg de protéines.

La présente invention a encore pour but de résoudre ces nouveaux problèmes techniques avec la mise en oeuvre d'un procédé de fabrication particulièrement simple, reproductible, utilisable à l'échelle industrielle.

La présente invention fournit pour la première fois une solution satisfaisante à ces problèmes techniques qui la rend utilisable à l'échelle industrielle, en permettant de traiter des grandes quantités sans limitation de volume contrairement aux techniques antérieures, qui étaient limitées aux laboratoires.

En outre, l'invention réalise une inactivation virale, qui n'est pas prévue dans les documents antérieurs.

Ainsi, selon un premier aspect, la présente invention fournit un procédé de fabrication du Facteur antihémophilique (FVIIIc) ayant une très haute pureté, dépourvu en majeure partie du Facteur Willebrand, comprenant une étape de purification par chromatographie d'échange d'ions à l'aide d'une colonne de chromatographie contenant un gel, comprenant une étape d'adsorption du Facteur antihémophilique sur le gel de ladite colonne et une étape de désorption du Facteur antihémophilique purifié que l'on recueille, caractérisé en ce qu'on prépare une première solution d'élution tamponnée, sans ajout de carbohydrate, qui présente une force ionique capable de séparer le Facteur VIIIc de la majeure partie du Facteur Willebrand ainsi que de ses principaux contaminants plasmatiques ; on prépare une solution brute du Facteur VIIIc à partir d'un cryoprécipité, sans ajout de carbohydrate, qui présente une force ionique sensiblement identique à celle de la première solution d'élution tamponnée ; on adsorbe sélectivement le Facteur VIIIc essentiellement sans Facteur Willebrand sur le gel de la colonne en éluant ladite solution brute du Facteur VIIIc avec ladite première solution d'élution ; et on désorbe le Facteur VIIIc adsorbé sur le gel de la colonne grâce à la première solution d'élution en utilisant une deuxième solution d'élution tamponnée de force ionique plus élevée que la première solution d'élution, en obtenant ainsi une solution purifiée de Facteur VIIIc de très haute pureté, dépourvue en majeure partie du Facteur Willebrand ainsi que des principaux contaminants plasmatiques.

Selon une variante de réalisation avantageuse du procédé selon l'invention, on procède ensuite à une diafiltration contre une troisième solution tampon, de préférence permettant l'injection intraveineuse. Selon une autre caractéristique avantageuse, on concentre la solution purifiée du Facteur VIIIc et encore de préférence on lyophilise.

Selon une variante de réalisation du procédé selon l'invention, on prépare la solution brute à purifier de Facteur VIIIc par mise en solution d'un cryoprécipité, extrait de plasma humain.

Selon une autre caractéristique avantageuse du procédé selon l'invention, on traite la solution obtenue par dissolution du cryoprécipité dans de l'eau avantageusement additionnée d'héparine avec de l'hydroxyde d'aluminium. La proportion d'incorporation d'hydroxyde d'aluminium n'est pas critique et peut varier dans de larges limites. On préfère cependant utiliser une proportion supérieure à 10 % en volume par rapport au volume total de la solution contenant le Facteur VIIIc. Des proportions actuellement préférées sont de l'ordre de 15 % en volume.

Avantageusement on procède ensuite à une centrifugation pour séparer l'hydroxyde d'aluminium de la solution de Facteur VIIIc débarrassée ainsi des Facteurs vitamine K dépendants. Selon une variante de réalisation avantageuse du procédé selon l'invention, on peut ensuite inactiver viralement la solution contenant le Facteur VIIIc à l'aide d'une solution tampon contenant un mélange solvant/détergent comme décrit dans le document EP-A-0 131 740 ou US-A-4 540 573.

La solution brute à purifier de Facteur VIIIc est de préférence diafiltrée contre la première solution tampon servant à l'élution de la colonne, avant de passer à l'étape d'adsorption sur la colonne.

Selon une caractéristique avantageuse du procédé selon l'invention, le gel de purification utilisé dans la colonne de chromatographie par échange d'ions est un gel présentant les caractéristiques essentielles suivantes :
- un gel d'échange d'ions de type anionique à base d'agarose réticulé en particulier à environ 6 % sous forme de billes. Ce gel est de préférence de type amine quaternaire, notamment en bout d'un petit bras espaceur, par exemple de type alkylène en C₁-C₆, relié aux billes d'agarose.
- un gel qui correspond à ces caractéristiques et qui est disponible commercialement est connu sous le nom commercial Q Sépharose fast flow® et commercialisé par la société suédoise Pharmacia, dont les billes ont un diamètre de 45-165 µm à l'état humide.

Selon une autre caractéristique avantageuse du procédé selon l'invention, la première solution d'élution tampon présente la composition suivante :
- NaCl 250 mmol/l,
- Tris 20 mmol/l,
- CaCl₂,2H₂O 10 mmol/l.

Cette composition est amenée à pH 6,6 avec de l'HCl concentré 12 N.

Selon une autre caractéristique avantageuse du procédé selon l'invention, la deuxième solution tampon servant à la désorption du Facteur VIIIc de la colonne présente la composition suivante :
- NaCl 700 mmol/l,
- Tris 20 mmol/l,
- CaCl₂,2H₂O 10 mmol/l.

Cette composition est également amenée à pH 6,6 avec de l'HCl concentré 12 N.

Selon encore une autre caractéristique avantageuse du procédé selon l'invention, la troisième solution tampon précitée utilisée pour la diafiltration du Facteur VIIIc purifié et élué présente la composition suivante :
- NaCl 100 mmol/l,
- Tris 20 mmol/l,
- CaCl₂,2H₂O 0,6 mmol/l,
- L-arginine 17 mmol/l,
- L-lysine-HCl 20 mmol/l.

Cette solution est également amenée à pH 7 avec de l'HCl concentré 12 N.

Selon une caractéristique avantageuse du procédé selon l'invention, avant de procéder à cette diafiltration avec la troisième solution tampon, on ajoute dans la solution de Facteur VIIIc purifiée éluée 17 mmol/l de L-arginine ainsi que 20 mmol/l de L-lysine-HCl par litre de solution.

Selon une caractéristique avantageuse du procédé de l'invention, on réalise les diafiltrations précitées avec des membranes de diafiltrations traitées avec un tampon bicarbonate 0,1 M à pH 9,6 contenant 30 g/l d'albumine humaine apyrogène et 0,1 % en volume d'agent émulsifiant.

En vue de la conservation de la solution de Facteur VIIIc éluée de très haute pureté ainsi obtenue, dépourvue en majeure partie de Facteur Willebrand, et présentant une activité antihémophilique A d'au moins 250 U.I./mg de protéines, on procède à une filtration stérilisante sur filtre stérilisant ayant une dimension de pore de diamètre 0,22 µm, puis on introduit cette solution de Facteur VIIIc purifiée, stérilisée, dans des flacons siliconés.

D'autres buts, caractéristiques et avantages l'invention apparaîtront clairement à la lumière de la description explicative qui va suivre faite en référence à un exemple de réalisation donné simplement à titre d'illustration et qui ne saurait donc en aucune façon limiter la portée de l'invention. Dans cet exemple, les pourcentages sont donnés en poids sauf indication contraire.

### Exemple de l'invention

On prend 3 kg de cryoprécipité de plasma sanguin humain, issu de 300 l de plasma humain, que l'on met en solution dans 3,2 fois son volume de solution d'eau osmosée, limulus négatif, additionnée d'héparine à raison de 3 U.I./ml de solution, à la température du laboratoire et sous agitation pendant 2 h de manière à réaliser une dissolution complète.

Après mesure du volume final, on additionne à cette solution de l'hydroxyde d'aluminium à raison de 15 % en volume par rappore au volume total de cette solution afin d'éliminer les Facteurs vitamine K dépendants. On laisse sous agitation pendant 5 min pour réaliser cette adsorption des Facteurs vitamine K dépendants sur l'hydroxyde d'aluminium.

On centrifuge à 6 000 G pour séparer l'hydroxyde d'aluminium de la solution.

Ensuite, on traite la solution contenant le Facteur VIIIc selon le procédé décrit dans le document EP-A-0 131 740 ou US-A-4 540 573 de manière à réaliser une inactivation virale.

Ensuite, on procède à une diafiltration de la solution contenant le Facteur VIIIc inactivé contre une première solution tampon qui sera ensuite utilisée pour l'équilibration et la première élution de la colonne de chromatographie, ayant la composition suivante :
- NaCl 250 mmol/l,
- Tris 20 mmol/l,
- CaCl₂,2H₂O 10 mmol/l.

Cette solution a été amenée à pH 6,6 avec de l'HCl concentré 12 N.

Cette diafiltration est réalisée avec deux fois le volume de la solution de Facteur VIIIc inactivée en éliminant ainsi la majeure partie des produits ayant servi à l'inactivation virale. Cette première membrane servant à la diafiltration est une membrane millipore référence 4PTHK à pouvoir de coupure à 100 000. On obtient ainsi une solution de Facteur VIIIc inactivée, diafiltrée et concentrée à 10 l de solution totale.

On vérifie que cette solution concentrée à 10 l présente sensiblement la même force ionique que la première solution tampon d'élution de la colonne de chromatographie. La colonne de chromatographie est par exemple une colonne de 25 cm de hauteur sur un diamètre de 40 cm dans laquelle on introduit 32 l de gel de chez Pharmacia Q-Sépharose fast flow®.

Le gel a été équilibré par la première solution d'élution avant de procéder à l'injection des 10 l de solution de Facteur VIIIc à purifier à raison de 25 l à l'heure pour l'injection que l'on suit par une injection au même débit de la première solution tampon d'élution jusqu'à ce que l'on n'observe plus en sortie de colonne de pic protéique, par vérification avec un spectrophotomètre à 280 nm, correspondant principalement au Facteur Willebrand, au fibrinogène, à l'albumine et aux Ω-globulines que l'on désire éliminer.

Après cette élution de la colonne avec la première solution tampon d'élution, on utilise comme solution d'élution une deuxième solution tampon d'élution ayant une force ionique plus élevée, ayant la composition suivante :
- NaCl 700 mmol/l,
- Tris 20 mmol/l,
- CaCl₂,2H₂O 10 mmol/l.

qui a été amenée à pH 6,6 par addition d'HCl concentré 12 N.

On réalise cette élution avec la deuxième solution tampon jusqu'à observation du pic correspondant au Facteur VIIIc, au même débit que précédemment. Cette observation est faite en modifiant la sensibilité du spectrophotomètre, comme cela est bien apparent à l'homme de l'art.

On observera que dans le pic initial non retenu qui contenait notamment tous les contaminants et parmi eux le Facteur Willebrand, on récupère un volume de 80 à 100 l de solution que l'on met de côté, qui est peut être traité pour la séparation sous forme purifiée du Facteur Willebrand, qui a un intérêt thérapeutique bien connu à l'homme de l'art dans le cadre de la maladie Willebrand.

Par ailleurs, la solution de Facteur VIIIc obtenue après élution présente un volume total de 40 à 50 l dans laquelle le Facteur VIIIc est présent en très faible proportion c'est-à-dire entre 0,5 et 1 U.I./ml.

A cette solution diluée ainsi obtenue, on ajoute 17 mmol/l de L-arginine et 20 mmol/l de L-lysine-HCl.

Par la suite, on procède à une concentration de la solution en diafiltrant contre une troisième solution tampon présentant la composition suivante :
- NaCl 100 mmol/l,
- Tris 20 mmol/l,
- CaCl₂,2H₂O 0,6 mmol/l,
- L-arginine 17 mmol/l,
- L-lysine-HCl 20 mmol/l,

cette solution ayant été amenée à un pH égal à 7 par de l'HCl concentré 12 N.

Cette manière de procéder permet de diminuer la proportion de sodium à 100 mmol/l et à concentrer à 30 U.I./ml le Facteur VIIIc. La membrane de diafiltration qui est utilisée à cette étape est une membrane millipore, référence 4PTTK, ayant un pouvoir de coupure à 30 000.

Il est à noter qu'il est très important de traiter auparavant toutes les membranes de diafiltration en les rinçant abondamment à de l'eau osmosée limulus négatif. On a préparé par ailleurs 5 l de tampon bicarbonate 0,1 M à pH 9,6 contenant 30 g/l d'albumine humaine apyrogène et 0,1 % en volume d'agent émulsifiant, par exemple, Tween 80® que l'on injecte au travers de ces membranes grâce à une pompe ASTI® à piston en Téflon et un corps en Pyrex, en circuit fermé pendant 2 h. On rince ensuite pendant 1 h à l'eau osmosée limulus négatif puis l'on passe 20 l de la deuxième solution tampon précitée. On vérifie que le taux de protéine à la sortie de la membrane de diafiltration est nul par un dosage de protéine en utilisant la microméthode de Marion M. Bradford décrite dans ANALYTICAL BIOCHEMISTRY 72, 248-254 (1976).

On procède ensuite à une filtration stérilisante de la solution du Facteur VIIIc sur une membrane stérilisante par exemple de chez millipore référence Millidisk à diamètre de pore de 0,22 µm.

On obtient ainsi une solution de Facteur VIIIc de très haute pureté, stérilisée, que l'on peut répartir dans des flacons siliconés pour le stockage des produits, notamment en vue de la lyophilisation que l'on peut réaliser ensuite de manière tout à fait classique. On obtient ainsi un Facteur VIIIc à 30 U.I./ml, avec une activité spécifique d'au moins 250 U.I./mg de protéines.

Ce Facteur VIIIc à avantageusement lyophiliser constitue ainsi une composition pharmaceutique de très grande valeur qui permet de n'injecter au patient souffrant d'hémophilie que les quantités de produit nécessaires à traiter leur hémophilie, ce qui constitue un progrès technique déterminant, tout à fait inattendu pour l'homme de l'art.

Il est encore à observer que ce produit présente une très grande stabilité dans le temps. En particulier, l'activité du Facteur VIIIc baisse de moins de 10 % après reprise du lyophilisat au bout de 24 h.

En outre, avec le procédé selon l'invention, on obtient d'excellents rendements au niveau industriel, qui sont de l'ordre de 50 à 55 % par rapport au cryoprécipité dissous de départ.

## Revendications (Revendications pour l'(les) Etat(s) contractant(s) suivant(s): DE, GB, IT, NL, SE, CH, LI, BE, AT, LU, DK, ES, GR)

1. Procédé de fabrication du Facteur antihémophilique (FVIIIc) ayant une très haute pureté, dépourvu en majeure partie du Facteur Willebrand, comprenant une étape de purification par chromatographie d'échange d'ions à l'aide d'une colonne de chromatographie comprenant un gel, comprenant une étape d'adsorption du Facteur antihémophilique sur le gel de ladite colonne et une étape de désorption du Facteur antihémophilique purifié que l'on recueille, caractérisé en ce qu'on prépare une première solution d'élution tamponnée, sans ajout de carbohydrate, qui présente une force ionique capable de séparer le Facteur VIIIc de la majeure partie du Facteur Willebrand ainsi que de ses principaux contaminants plasmatiques ; on prépare une solution brute du Facteur VIIIc à partir d'un cryoprécipité, sans ajout de carbohydrate, qui présente une force ionique sensiblement identique à celle de la première solution d'élution tamponnée ; on adsorbe sélectivement le Facteur VIIIc, essentiellement sans le Facteur Willebrand, sur le gel de la colonne en éluant ladite solution brute du Facteur VIIIc avec ladite première solution d'élution ; et on désorbe le Facteur VIIIc adsorbé sur le gel de la colonne grâce à la première solution d'élution en utilisant une deuxième solution d'élution tamponnée de force ionique plus élevée que la première solution d'élution, en obtenant ainsi une solution purifiée de Facteur VIIIc de très haute pureté, dépourvu en majeure partie du Facteur Willebrand ainsi que des principaux contaminants plasmatiques.

2. Procédé selon la revendication 1, caractérisé en ce qu'on procède à une diafiltration contre une troisième solution tampon, de préférence permettant l'injection intraveineuse.

3. Procédé selon la revendication 1 ou 2, caractérisé en ce qu'on concentre la solution purifiée du Facteur VIIIc qui est de préférence lyophilisée.

4. Procédé selon l'une des revendications 1 à 3, caractérisé en ce qu'on prépare la solution brute à purifier de Facteur VIIIc par mise en solution d'un cryoprécipité d'un extrait de plasma humain.

5. Procédé selon l'une des revendications 1 à 4, caractérisé en ce qu'on traite la solution obtenue par dissolution du cryoprécipité dans de l'eau avantageusement additionnée d'héparine, avec de l'hydroxyde d'aluminium.

6. Procédé selon la revendication 5, caractérisé en ce que la proportion d'incorporation d'hydroxyde d'aluminium est supérieure à 10 % en volume par rapport au volume total de la solution contenant le Facteur VIIIc, et encore de préférence et de l'ordre de 15 % en volume.

7. Procédé selon la revendication 5 ou 6, caractérisé en ce qu'on procède à une centrifugation pour séparer l'hydroxyde d'aluminium de la solution de Facteur VIIIc débarrassée ainsi des Facteurs vitamine K dépendants.

8. Procédé selon l'une des revendications 5 à 7, caractérisé en ce qu'on inactive viralement la solution contenant le Facteur VIIIc à l'aide d'une solution tampon contenant un mélange solvant/ détergent.

9. Procédé selon l'une des revendications 1 à 8, caractérisé en ce que la solution brute purifiée de Facteur VIIIc est diafiltrée contre la première solution tampon précitée servant à l'élution de la colonne, avant de passer à l'étape d'adsorption sur la colonne.

10. Procédé selon l'une des revendications 1 à 9, caractérisé en ce que le gel de purification utilisé dans la colonne de chromatographie par échange d'ions est un gel présentant les caractéristiques essentielles suivantes :
- un gel d'échange d'ions de type anionique à base d'agarose réticulé en particulier à environ 6 % sous forme de billes, de préférence du type amine quaternaire, notamment en bout d'un petit bras espaceur, par exemple de type alkylène en C₁-C₆ relié aux billes d'agarose.
- de préférence, ce gel est un gel connu sous le nom commercial Q-Sépharose fast flow® (PHARMACIA) dont les billes ont un diamètre de 45-165 µm à l'état humide.

11. Procédé selon l'une des revendications 1 à 10, caractérisé en ce que la première solution d'élution tampon précitée présente la composition suivante :
- NaCl 250 mmol/l,
- Tris 20 mmol/l,
- CaCl₂,2H₂O 10 mmol/l,
cette composition étant amenée à pH 6,6 avec le l'HCl concentré 12 N.

12. Procédé selon l'une des revendications 1 à 11, caractérisé en ce que la deuxième solution tampon précitée servant à la désorption du Facteur VIIIc de la colonne présente la composition suivante :
- NaCl 700 mmol/l,
- Tris 20 mmol/l,
- CaCl₂,2H₂O 10 mmol/l,
cette composition étant amenée à pH 6,6 avec de l'HCl concentré 12 N.

13. Procédé selon l'une des revendications 2 à 12, caractérisé en ce que la troisième solution tampon précitée utilisée pour la diafiltration du Facteur VIIIc purifié et élué présente la composition suivante :
- NaCl 100 mmol/l,
- Tris 20 mmol/l,
- CaCl₂,2H₂O 0,6 mmol/l,
- L-arginine 17 mmol/l,
- L-lysine-HCl 20 mmol/l.
cette solution étant amenée à pH 7 avec de l'HCl concentré 12 N.

14. Procédé selon l'une des revendications 2 à 13, caractérisé en ce que, avant de procéder à cette diafiltration avec la troisième solution tampon, on ajoute dans la solution de Facteur VIIIc purifiée éluée 17 mmol/l de L-arginine ainsi que 20 mmol/l de L-lysine-HCl par litre de solution.

15. Procédé selon l'une des revendications 1 à 14, caractérisé en ce qu'on procède à une filtration stérilisante sur filtre stérilisant ayant une dimension de pore de diamètre 0,22 µm de la solution de Facteur VIIIc éluée de très haute pureté, puis on introduit cette solution de Facteur VIIIc purifiée, stérilisée, dans des flacons siliconés.

16. Procédé selon l'une des revendications 2 à 15, caractérisé en ce qu'on réalise les diafiltrations avec des membranes de diafiltration traitées avec un tampon bicarbonate 0,1 M à pH 9,6 contenant 30 g/l d'albumine humaine apyrogène et 0,1 % en volume d'agent émulsifiant.

17. Procédé de préparation d'une composition pharmaceutique, caractérisé en qu'on utilise comme principe actif un Facteur VIIIc de très haute pureté tel qu'obtenu par le procédé selon l'une quelconque des revendications 1 à 16, de préférence sous forme lyophilisée, permettant de préparer extemporanément une préparation injectable.

## Claims (Claims for the following Contracting State(s): DE, GB, IT, NL, SE, CH, LI, BE, AT, LU, DK, ES, GR.)

1. A process for the preparation of very high-purity Antihemophilic Factor (FVIIIc) devoid of the bulk of the Willebrand Factor, comprising a step for purification by ion exchange chromatography with the aid of a chromatography column containing a gel, said purification step comprising a step for adsorption of the Antihemophilic Factor on the gel in said column and a step for desorption of the purified Antihemophilic Factor, which is collected, wherein a first buffered eluting solution is prepared which has an ionic strength capable of separating Factor VIIIc from the bulk of the Willebrand Factor and its main plasma contaminants, a crude solution of Factor VIIIc is prepared from a cryoprecipitate, said crude solution having a substantially identical ionic strength to that of the first buffered eluting solution, the Factor VIIIc essentially devoid of Willebrand Factor is selectively adsorbed on the gel in the column, said crude solution of Factor VIIIC being eluted with said first eluting solution, and the Factor VIIIc adsorbed on the gel in the column by means of the first eluting solution is desorbed using a second buffered eluting solution of higher ionic strength than the first eluting solution, thus giving a purified solution of Factor VIIIc of very high purity which is devoid of the bulk of the Willebrand Factor and the main plasma contaminants.

2. Process according to claim 1, characterized in that the diafiltration is carried out against a third buffer solution, preferably permitting intravenous injection.

3. Process according to claim 1, characterized in that the purified solution of Factor VIIIc is concentrated and preferably lyophilized.

4. Process according to claim 1, characterized in that the crude solution of Factor VIIIc to be purified is prepared by dissolving a cryoprecipitate of a human plasma extract.

5. Process according to one of claims 1 to 4, characterized in that the solution obtained by dissolving the cryoprecipitate in water to which heparin has advantageously been added is treated with aluminum hydroxide.

6. Process according to claim 5, characterized in that the proportion in which aluminum hydroxide is incorporated is greater than 10% by volume, relative to the total volume of the solution containing Factor VIIIc, and preferably of the order of 15% by volume.

7. Process according to claim 5 or 6, characterized in that the centrifugation is carried out in order to separate the aluminum hyroxide from the solution of Factor VIIIc, from which the vitamin K dependent factors are thereby removed.

8. Process according to one of claims 5 to 7, characterized in that the solution containing Factor VIIIc is virally inactivated with the aid of a buffer solution containing a solvent/detergent mixture.

9. Process according to one of claims 1 to 8, characterized in that the purified crude solution of Factor VIIIc is subjected to diafiltration against the first buffer solution mentioned above, used to elute the column, before passing on to the adsorption step on the column.

10. Process according to one of claims 1 to 9, characterized in that the purification gel used in the ion exchange chromatography column is a gel having the following essential characteristics:
- an ion exchange gel of the anionic type based on agarose crosslinked in particular to the extent of about 6%, in the form of beads, and preferably of the quaternary amine type, especially at the end of a short spacer arm, for example of the C₁-C₆ alkylene type linked to the agarose beads;
- this gel is preferably a gel known under the tradename Q-Sepharose fast flow® (PHARMACIA), the beads having a diameter of 45-165 um in the wet state.

11. Process according to one of claims 1 to 10, characterized in that the first buffered eluting solution mentioned above has the following composition:
- NaCl 250 mmol/l
- Tris 20 mmol/l
- CaCl₂.2H₂O 10 mmol/l
this composition being brought to pH 6.6 with 12 N concentrated HCl.

12. Process according to one of claims 1 to 11, characterized in that the second buffer solution mentioned above, used for desorption of the Factor VIIIc from the column, has the following composition:
- NaCl 700 mmol/l
- Tris 20 mmol/l
- CaCl₂.2H₂O 10 mmol/l
this composition being brought to pH 6.6 with 12 N concentrated HCl.

13. Process according to one of claims 2 to 12, characterized in that the third buffer solution mentioned above, used for diafiltration of the purified and eluted Factor VIIIc, has the following composition:
- NaCl 100 mmol/l
- Tris 20 mmol/l
- CaCl₂.2H₂O 0.6 mmol/l
- L-arginine 17 mmol/l
- L-lysine-HCl 20 mmol/l
this solution being brought to pH 7 with 12 N concentrated HCl.

14. Process according to one of claims 2 to 13, characterized in that, before this diafiltration is carried out with the third buffer solution, 17 mmol/l of L-arginine and 20 mmol/l of L-lysine-HCl per liter of solution are added to the purified and eluted solution of Factor VIIIc.

15. Process according to one of claims 1 to 14, characterized in that the very high-purity eluted solution of Factor VIIIc is subjected to sterilizing filtration on a sterilizing filter with a pore diameter of 0.22 µm, and this purified and sterilized solution of Factor VIIIc is then introduced into silicon-treated flasks.

16. Process according to one of claims 2 to 15, characterized in that the diafiltrations are performed with diafiltration membranes treated with a 0.1 M bicarbonate buffer at pH 9.6, containing 30 g/l of non-pyrogenic human albumin and 0.1% by volume of emulsifier.

17. Process for the preparation of a pharmaceutical composition, characterized in that a very high-purity factor VIIIc such as obtained by the process according to any one of claims 1 to 16, is used as active principle, preferably in lyophilized form, permitting the extemporaneous preparation of an injectable composition.

## Patentansprüche (Patentansprüche für folgende(n) Vertragsstaat(en): DE, GB, IT, NL, SE, CH, LI, BE, AT, LU, DK, ES, GR)

1. Verfahren zur Herstellung des antihämophilen Faktors (FVIIIc) in sehr hoher Reinheit, aus dem der größte Teil des von-Willebrand-Faktors entfernt ist, das einen Reinigungsschritt durch Ionenaustauschchromatographie mit Hilfe einer ein Gel enthaltenden Chromatographiesäule umfaßt , der einen Adsorptionsschritt des antihämophilen Faktors am Gel der Säule und einen Desorptionsschritt des gereinigten antihämophilen Faktors, den man sammelt, umfaßt, dadurch gekennzeichnet, daß man eine erste Pufferlösung zur Elution ohne Kohlenhydratzusatz herstellt, die eine zur Trennung des Faktors VIIIc vom Großteil des von Willebrand-Faktors und von den Hauptplasmaverunreinigungen ausreichende Ionenstärke besitzt, man eine Rohlösung des Faktors VIIIc, ausgehend von einem Cryopräcipitat, ohne Kohlenhydratzusatz herstellt, die ungefähr die gleiche Ionenstärke wie die erste Pufferlösung zur Elution besitzt, man selektiv den Faktor VIIIc, im wesentlichen ohne den von Willebrand-Faktor, auf dem Gel der Säule adsorbiert, indem die Rohlösung des Faktors VIIIc mit der ersten Elutionslösung eluiert wird, und man den am Gel der Säule mit Hilfe der ersten Elutionslösung adsorbierten Faktor VIIIc unter Verwendung einer zweiten Pufferlösung zur Elution mit einer höheren Ionenstärke als der ersten Elutionslösung desorbiert, wobei man eine gereinigte Lösung des Faktors VIIIc in sehr hoher Reinheit gewinnt, aus dem der größte Teil des von Willebrand-Faktors und die Hauptplasmaverunreinigungen entfernt sind.

2. Verfahren nach Anspruch 1, dadurch gekennzeichnet, daß man eine Diafiltration gegen eine dritte Pufferlösung durchführt, die vorzugsweise die intravenöse Injektion erlaubt.

3. Verfahren nach Anspruch 1 oder 2, dadurch gekennzeichnet, daß man die gereinigte Lösung des Faktors VIIIc, die vorzugsweise lyophilisiert ist, aufkonzentriert.

4. Verfahren nach einem der Ansprüche 1 bis 3, dadurch gekennzeichnet, daß man die zu reinigende Rohlösung des Faktors VIIIc durch Auflösen eines Cryopräcipitats eines Extraktes von Humanplasma herstellt.

5. Verfahren nach einem der Ansprüche 1 bis 4, dadurch gekennzeichnet, daß man die durch Auflösung des Cryopräcipitats in vorteilhaft heparinhaltigem Wasser erhaltene Lösung mit Aluminiumhydroxid behandelt.

6. Verfahren nach Anspruch 5, dadurch gekennzeichnet, daß die Zugabemenge an Aluminiumhydroxid über 10 Vol.-% in bezug auf das Gesamtvolumen der den Faktor VIIIc enthaltenden Lösung und vorzugsweise in der Größenordnung von 15 Vol.-% liegt.

7. Verfahren nach Anspruch 5 oder 6, dadurch gekennzeichnet, daß man eine Zentrifugierung durchführt, um das Aluminiumhydroxid aus der so von den Vitamin K-abhängigen Faktoren befreiten Lösung des Faktors VIIIc abzutrennen.

8. Verfahren nach einem der Ansprüche 5 bis 7, dadurch gekennzeichnet, daß man die den Faktor VIIIc enthaltende Lösung mit Hilfe einer ein Lösungsmittel/Detergens-Gemisch enthaltenden Pufferlösung virusinaktiviert.

9. Verfahren nach einem der Ansprüche 1 bis 8, dadurch gekennzeichnet, daß die gereinigte Rohlösung des Faktors VIIIc vor dem Adsorptionsschritt an der Säule gegen die vorher erwähnte erste Pufferlösung, die zur Elution der Säule dient, diafiltriert wird.

10. Verfahren nach einem der Ansprüche 1 bis 9, dadurch gekennzeichnet, daß das in der Säule zur Ionenaustauschchromatographie verwendete Reinigungsgel ein Gel mit den folgenden Haupteigenschaften ist:
- ein anionisches Ionenaustauschgel auf der Basis von vernetzter Agarose, insbesondere mit ungefähr 6 % in Form von Kügelchen, vorzugsweise auf der Basis von quaternärem Amin, insbesondere am Ende eines kleinen Spacers, beispielsweise des C₁-C₆-Alkylentyps, der an die Agarosekügelchen gebunden ist.
- vorzugsweise ist das Gel ein unter dem Handelsnamen Q-Sépharose fast flow® (PHARMACIA) bekanntes Gel mit Kügelchen eines Durchmessers von 45 - 165 µm im feuchten Zustand.

11. Verfahren nach einem der Ansprüche 1 bis 10, dadurch gekennzeichnet, daß die vorgenannte erste Pufferlösung zur Elution die folgende Zusammensetzung besitzt:
- NaCl 250 mmol/l,
- Tris 20 mmol/l,
- CaCl₂·2H₂O 10 mmol/l,
wobei diese Zusammensetzung mit konzentrierter 12 N HCl auf einen pH-Wert von 6,6 eingestellt ist.

12. Verfahren nach einem der Ansprüche 1 bis 11, dadurch gekennzeichnet, daß die vorgenannte zweite Pufferlösung zur Desorption des Faktors VIIIc von der Säule die folgende Zusammensetzung besitzt:
- NaCl 700 mmol/l,
- Tris 20 mmol/l,
- CaCl₂·2H₂O 10 mmol/l,
wobei diese Zusammensetzung mit konzentrierter 12 N HCl auf einen pH-Wert von 6,6 eingestellt ist.

13. Verfahren nach einem der Ansprüche 2 bis 12, dadurch gekennzeichnet, daß die zur Diafiltration des gereinigten und eluierten Faktors VIIIc verwendete vorgenannte dritte Pufferlösung folgende Zusammensetzung besitzt:
- NaCl 100 mmol/l,
- Tris 20 mmol/l,
- CaCl₂·2H₂O 0,6 mmol/l,
- L-Arginin 17 mmol/l,
- L-Lysin·HCl 20 mmol/l,
wobei diese Lösung mit konzentrierter 12 N HCl auf einen pH-Wert von 7 eingestellt ist.

14. Verfahren nach einem der Ansprüche 2 bis 13, dadurch gekennzeichnet, daß man vor der Diafiltration mit der dritten Pufferlösung der gereinigten und eluierten Lösung des Faktors VIIIc 17 mmol/l L-Arginin und 20 mmol/l L-Lysin·HCl pro Liter Lösung zusetzt.

15. Verfahren nach einem der Ansprüche 1 bis 14, dadurch gekennzeichnet, daß man eine Filtration zur Sterilisierung der eluierten Lösung des Faktors VIIIc sehr hoher Reinheit über einem sterilisierenden Filter mit einem Porendurchmesser von 0,22 µm durchführt, und man dann diese gereinigte, sterilisierte Lösung des Faktors VIIIc in siliconbeschichtete Flaschen abfüllt.

16. Verfahren nach einem der Ansprüche 2 bis 15, dadurch gekennzeichnet, daß man die Diafiltrationen mit Membranen zur Diafiltration durchführt, die mit einem 30 g/l apyrogenes Humanalbumin und 0,1 Vol.-% eines Emulgiermittels enthaltenden Bicarbonatpuffer (0,1 M, pH 9,6) behandelt sind.

17. Verfahren zur Herstellung einer pharmazeutischen Zusammensetzung, dadurch gekennzeichnet, daß man als Wirkstoff einen Faktor VIIIc sehr hoher Reinheit verwendet, wie er mit dem Verfahren nach einem der Ansprüche 1 bis 16 erhalten ist, vorzugsweise in lyophilisierter Form, die die Herstellung einer injizierbaren Präparation unmittelbar vor der Behandlung erlaubt.
